# EUROPÄISCHE PATENTSCHRIFT

(11) **EP 4 347 204 B1**
(45) Veröffentlichungstag und Bekanntmachung des Hinweises auf die Patenterteilung: **06.08.2025**
(21) Anmeldenummer: 22733327.5
(22) Anmeldetag: 02.06.2022
(51) Int. Cl.: B27G 15/00, A61B 17/16

(54) **BOHRNADEL, BOHRWIDERSTANDSMESSGERÄT UND DESSEN VERWENDUNG ZUR HOLZBESCHAFFENHEITSUNTERSUCHUNG**
DRILLING NEEDLE, DRILLING RESISTANCE METER AND ITS USE IN WOOD TEXTURE TESTING
AIGUILLE DE PERÇAGE, APPAREIL DE MESURE DE LA RÉSISTANCE DE PERÇAGE ET SON UTILISATION POUR L'EXAMEN DE LA QUALITÉ DU BOIS

(30) Priorität: 04.06.2021 DE 202021103038 U
(43) Veröffentlichungstag der Anmeldung: 10.04.2024
(73) Patentinhaber: IML Instrumenta Mechanik Labor GmbH, 69168 Wiesloch (DE)
(72) Erfinder: HUNGER, Erich, 76133 Karlsruhe (DE); HUNGER, Sebastian, 69181 Leimen (DE); HUNGER, Fabian, 69181 Leimen (DE)
(74) Vertreter: mepat Patentanwälte
(86) Internationale Anmeldenummer: PCT/EP2022/065108
(87) Internationale Veröffentlichungsnummer: WO 2022/253976

(56) Entgegenhaltungen:
- WO-A1-98/05459
- JP-A- 2002 039 929
- US-A1- 2006 083 595

## Beschreibung

Die Erfindung betrifft eine Bohrnadel gemäß dem Oberbegriff des Anspruchs 1 und dessen Verwendung zur Beschaffenheitsuntersuchung von Holzobjekten. Ein solches Bohrnadel ist aus dem Dokument DE 10 2009 013069 A1 bekannt.

Aus dem Stand der Technik ist bekannt, Baumstämme und Holzmasten - mithin lebende und tote Holzobjekte - auf mögliche vorliegende Defekte wie Fäulnis oder ähnliches zu untersuchen. In jedem Falle ist dabei eine minimalinvasive Untersuchung wünschenswert, da durch die Untersuchung kein Schaden verursacht werden soll. Für diese Untersuchungen werden Bohrwiderstandsmessgeräte eingesetzt. Ein solches Bohrwiderstandsmessgerät ist beispielsweise aus DE 10 2013 001 711 A1 bekannt. In diesen Bohrwiderstandsmessgeräten werden für den überwachten Bohrwiderstandsmessvorgang dünne Bohrnadeln verwendet. Dabei wird die Bohrnadel mit hoher Drehzahl rotiert und axial vorgeschoben, wobei die Leistungsaufnahme des Nadelantriebs ein Maß für den Eindring- bzw. Bohrwiderstand ist, sodass ein Bohrwiderstandsprofil über den axialen Vorschubweg der Bohrnadel aufnehmbar ist.

Es ist bekannt, diese Bohrnadeln während des Bohrvorgangs zentriert zu führen, d. h. gegen eine Bewegung quer zur Bohrrichtung abzustützen. So ist aus DE 100 31 395 A1 ein Spezial-Becher-Teleskop bekannt, das die Bohrnadel in Durchtrittsöffnungen an den Becherböden führt und einfahrbar ist, indem die kleineren Becher in den nächst größeren hineinfahren. Die Bohrnadel, die üblicherweise aus einem langen Schaft mit einem Bohrnadelkopf am Ende, der eine Bohrspitze oder Schneide aufweist, ist nicht näher beschrieben.

Eine Vorrichtung zur Holzprüfung ist auch aus DE 41 22 494 B4 bekannt. Auch diese Vorrichtung weist zylindrische Teleskophülsen auf, in denen die Bohrnadel mittig geführt wird. Der Kopf der gezeigten Bohrnadel ist abgeflacht mit einer verbreiterten Bohrspitze, sodass der Rotationsdurchmesser des Bohrnadelkopfes größer ist als der Schaftdurchmesser.

Eine Nadel, die ebenfalls einen breiteren Kopf als Schaft zeigt, ist auch in der DE 44 38 383 C2 zu sehen: Der Nadelschaft geht in eine breite Schneide mit einem spitzen Dorn über.

Der Nadelkopf der Bohrnadel aus DE 10 2009 013 069 A1 soll eine gleichmäßige Rotation der Nadel bereitstellen und das abgespante Holzmaterial in Richtung des Nadelschafts verschieben. Dazu weist der Nadelkopf am Ende eines abgeflachten Keilabschnitts auch eine breite Schneide auf, deren Rotationsdurchmesser größer ist als der Schaftdurchmesser, wobei die Schneidkante im rechten Winkel zu der Rotationsachse der Bohrnadel verläuft. Dabei ist der Winkel, den der Keilabschnitt zu einer Ebene aufspannt, die durch die Rotationsachse und die Kante definiert wird, kleiner als der Winkel, den die Schneidflächen der Schneide zu der Ebene aufspannen.

Die bekannten Bohrnadeln eignen sich insbesondere für einen Eindringwinkel horizontal und im rechten Winkel zur Objektachse. Da Fäule bei Holzmasten, deren Fußabschnitt sich im Erdboden befindet, jedoch meist ca. 10 cm unterhalb der Erdgleiche sitzt, sind zur Mastkontrolle Bohrungen in einem Winkel von 15° bis 45° zur Erdgleiche unerlässlich, um mit der Bohrnadel Fäulnisbereiche ohne Ausgrabung des Mastfußes erreichen zu können. Erdgleiche bezeichnet hierbei eine virtuelle Durchschnittsebene der Erdbodenoberfläche im Standbereich des Holzobjekts. Herkömmliche Bohrnadeln neigen bei von 90° zur Objektachse abweichenden Eindringwinkeln zum Abdriften, wobei die Nadel von der beabsichtigten axialen Vorschubrichtung abweicht, sodass kein geradliniger Bohrkanal entsteht, sondern ein Kanal, der ggf. sogar den Fäulnisbereich verfehlen kann.

Daher besteht das Erfordernis, eine Bohrnadel dahingehend zu verbessern, dass auch unter einem von 90° zur Objektachse abweichenden Winkel das Einführen der Bohrnadel in das Objekt zuverlässig durchgeführt wird, um insbesondere auch Objektabschnitte unterhalb der Erdgleiche untersuchen zu können.

Diese Aufgabe wird durch eine Bohrnadel mit den Merkmalen des Anspruchs 1 und durch ein Bohrwiderstandsmessgerät mit den Merkmalen des Anspruchs 9 gelöst.

Mit den Merkmalen des unabhängigen Anspruchs 10 wird eine Verwendung der Bohrnadel bzw. des Bohrwiderstandsmessgeräts zur Holzbeschaffenheitsuntersuchung offenbart.

Weiterbildungen der Bohrnadel sind in den Unteransprüchen ausgeführt.

Eine erste Ausführungsform der erfindungsgemäßen Bohrnadel bezieht sich auf eine Bohrnadel mit einem Bohrnadelkopf, der an einem Ende eines Bohrnadelschafts einen abgeflachten Keilabschnitt mit zwei diametralen Keilflächen und einen Schneidenabschnitt mit zwei Schneidflächen aufweist, die jeweils an eine der Keilflächen anschließen und an einer Kopfkante aneinander angrenzen, die im rechten Winkel zu einer Rotationsachse A des Bohrnadelschafts verläuft. Ferner weist der Keilabschnitt zwei diametrale Flankensegmente auf, die sich seitlich an die Keilflächen anschließen und abgerundet sind. Erfindungsgemäß weist der Bohrnadelkopf seitlich an die zwei Schneidflächen angrenzend jeweils eine Freischlifffläche auf, deren Ausbildung auf eine Rotationsrichtung der Bohrnadel abgestimmt ist. So bildet jede der beiden Freischliffflächen mit jeweils einer der Schneidflächen eine in Bezug auf die Rotationsrichtung vorlaufende Schneidkante, die sich von einer Kopfecke an der Kopfkante zu einer Schneidkantenecke an der Keilfläche erstreckt. Entsprechend bildet jede der beiden Freischliffflächen mit der jeweils anderen der Schneidflächen eine in Bezug auf die Rotationsrichtung nachlaufende Nebenkante, die sich von der jeweiligen Kopfecke an der Kopfkante zu einer Nebenkantenecke an der Keilfläche erstreckt. Dabei liegt ein Freischliffwinkel α, den eine virtuelle Gerade, die durch die Schneidkantenecke und die Nebenkantenecke verläuft, mit einer Ebene aufspannt, die sich durch die Rotationsachse A senkrecht zu der Kopfkante erstreckt, in einem Bereich von 6° bis 10°. Damit ist die nachlaufende Nebenkante kürzer als die vorlaufende Schneidkante. Daher hat die Freischlifffläche die Form eines unregelmäßigen Vierecks mit einer aufgrund des gerundeten Flankensegments abgerundeten Seite, wobei die vier Ecken die Kopfecke, Schneidkantenecke, Nebenkantenecke und eine weitere Ecke benachbart zu der Nebenkantenecke an der Kante zwischen Keilfläche und Flankensegment sind.

Nach einer weiteren Ausführungsform der erfindungsgemäßen Bohrnadel kann der Freischliffwinkel α bei 8° liegen.

Bei Verwendung der erfindungsgemäßen Bohrnadel mit der spezifischen Bohrnadelkopfgeometrie wird das Abdriften bzw. Nicht-Geradeaus-Laufen der Bohrnadel beim Eindringen, insbesondere unter einem von 90° zur Objektachse abweichenden Eindringwinkel, minimiert, da durch die Schneidkante mit der hinterschnittenen Freischlifffläche verhindert wird, dass die Bohrnadel bei einem von 90° zur Objektachse abweichenden Eindringen mit der Kopfkante die Jahresringe zuerst berührt. Damit ermöglicht die erfindungsgemäße Bohrnadel zuverlässig geradlinige Bohrungen insbesondere auch in einem Winkel von 15° bis 45° zur Erdgleiche.

Nach einer weiteren Ausführungsform der erfindungsgemäßen Bohrnadel kann ein Schneidenwinkel γ, den die Schneidkante in Bezug auf die Rotationsachse bzw. in Bezug auf eine Ebene, die senkrecht zur Kopfkante durch die Rotationsachse verläuft, aufspannt, in einem Bereich von 25° bis 35° liegen, bevorzugt beträgt er 30°.

Weitere Ausführungsformen der erfindungsgemäßen Bohrnadel können vorsehen, dass die Kopfkante eine Breite aufweist, die einen ersten Rotationsdurchmesser bereitstellt, der breiter ist als ein Durchmesser des Bohrnadelschafts, sodass die Späne in Richtung des Schafts verdrängt werden können. Dabei kann bevorzugt zusätzlich der Bohrnadelkopf in einem Übergangsbereich des Keilabschnitts mit dem Schneidenabschnitt, d. h. im Bereich der abgerundeten Seite der Freischlifffläche am Flankensegment, einen zweiten Rotationsdurchmesser aufweisen, der größer ist als der erste Rotationsdurchmesser, da durch die Freischlifffläche der Spanabtransport sichergestellt ist. Alternativ oder zusätzlich kann für eine gleichmäßige Rotation der Bohrnadel ein Winkel δ zwischen den beiden Keilflächen kleiner sein als ein Kopfkantenwinkel β zwischen den beiden Schneidflächen.

Der Kopfkantenwinkel β kann dabei in einem Bereich von 85° bis 95° liegen und bevorzugt 90° betragen, während der Winkel δ zwischen den beiden Keilflächen in einem Bereich von 10° bis 20° liegen und beispielsweise 15° betragen kann.

Nach einer weiteren Ausführungsform einer erfindungsgemäßen Bohrnadel kann vorgesehen sein, dass der Bohrnadelkopf bzw. die gesamte Bohrnadel aus einem Stahl gefertigt ist, der oberflächengehärtet oder durchgehärtet sein kann, wobei zumindest der Bohrnadelkopf, vorzugsweise die gesamte Bohrnadel, eine Oberflächenbeschichtung aufweist.

Eine geeignete Oberflächenbeschichtung kann nach einer weiteren Ausführungsform einer erfindungsgemäßen Bohrnadel durch eine Hartverchromung gebildet werden, alternativ kann die Beschichtung Nickel oder Polytetrafluorethylen aufweisen, die Beschichtung kann vorteilhaft eine Schichtdicke in einem Bereich von 8 bis 12 µm aufweisen. Alternativ kann die Bohrnadel, zumindest deren schneidendes Ende, auch durch Nitrieren gehärtet sein.

Nach einer beispielhaften Ausführungsform einer erfindungsgemäßen Bohrnadel kann eine Breite der Kopfkante in einem Bereich von 2,00 bis 2,50 mm liegen und bevorzugt 2,20 mm betragen. Der Bohrnadelkopf weist dabei eine maximale Breite in einem Übergangsbereich des Keilabschnitts mit dem Schneidenabschnitt im Bereich der Abrundung der Freischlifffläche zwischen Schneidkantenecke und Nebenkantenecke auf, wobei diese maximale Breite des Bohrnadelkopfs in einem Bereich von 2,25 bis 3,00 mm liegen kann und bevorzugt 2,75 mm beträgt. Die zu dieser beispielhaften Ausführungsform gehörende Höhe des Schneidenabschnitts, die durch den axialen Abstand der Kopfkante zu der Keilabschnittskante definiert wird, die sich zwischen Schneidfläche und Keilfläche von Schneidkantenecke zu Nebenkantenecke erstreckt, kann in einem Bereich von 0,30 bis 0,40 mm liegen und bevorzugt 0,35 mm betragen.

Generell können alle hölzernen Materialien mit der gleichen Nadel bearbeitet werden, bei besonders harten Hölzern kann vorteilhaft eine Bohrnadel mit gehärteter Schneide verwendet werden.

Ein erfindungsgemäßes Bohrwiderstandsmessgerät weist in einer ersten Ausführungsform eine Bohrnadel und ein Handgerät auf, das zumindest eine Antriebsvorrichtung mit einem Bohrfutter aufweist, in dem die Bohrnadel eingespannt ist, die eine erfindungsgemäße Bohrnadel ist. Weitere Ausführungsformen des erfindungsgemäßen Bohrwiderstandsmessgeräts beziehen sich darauf, dass das Handgerät eine Führungsvorrichtung zum geführten Einführen der Bohrnadel in ein zu untersuchendes Holzobjekt und/oder eine Bohrwiderstandserfassungseinrichtung aufweisen kann.

Eine erfindungsgemäße Verwendung eines erfindungsgemäßen Bohrwiderstandsmessgeräts mit einer erfindungsgemäßen Bohrnadel zur Beschaffenheitsuntersuchung eines Holzobjekts wie einem Mast oder Baumstamm sieht vor, dass beim Einführen der Bohrnadel in das Holzobjekt in einer axialen Vorschubrichtung, die beliebig in Bezug auf eine Objektachse oder eine Erdgleiche wählbar ist, ein geradliniger Bohrkanal in der axialen Vorschubrichtung erzeugt wird. Bevorzugt kann dabei das Holzobjekt einen Abschnitt unter der Erdgleiche aufweisen und der Bohrkanal sich von einer Eindringstelle oberhalb der Erdgleiche in den Abschnitt unterhalb der Erdgleiche erstrecken, wobei ein Eindringwinkel ε zwischen der axialen Vorschubrichtung und der Erdgleiche in einem Bereich von 15° bis 45° liegen kann.

Bei Verwendung einer erfindungsgemäßen Bohrnadel können somit insbesondere auch Abschnitte der Holzobjekte unterhalb der Erdgleiche untersucht werden, die bei einer Bohrstelle oberhalb der Erdgleiche einen Eindringwinkel von 15° bis 45° erforderlich machen, wobei anders als bei herkömmlichen Bohrnadeln ein genauer gradliniger Bohrkanal erzeugt wird mit der erfindungsgemäßen Bohrnadel.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig.** 1: eine perspektivische Ansicht des Bohrnadelkopfes einer Bohrnadel in einer erfindungsgemäßen Ausführungsform,
- **Fig. 2**: eine Draufsicht auf den Bohrnadelkopf aus Fig. 1,
- **Fig. 3**: eine erste Seitenansicht auf die Bohrnadel aus Fig. 1,
- **Fig. 4**: eine zweite Seitenansicht auf die Bohrnadel aus Fig. 1,
- **Fig. 5**: eine schematische Ansicht eines zu untersuchenden Holzobjekts mit einem erfindungsgemäßen Bohrwiderstandsmessgerät.

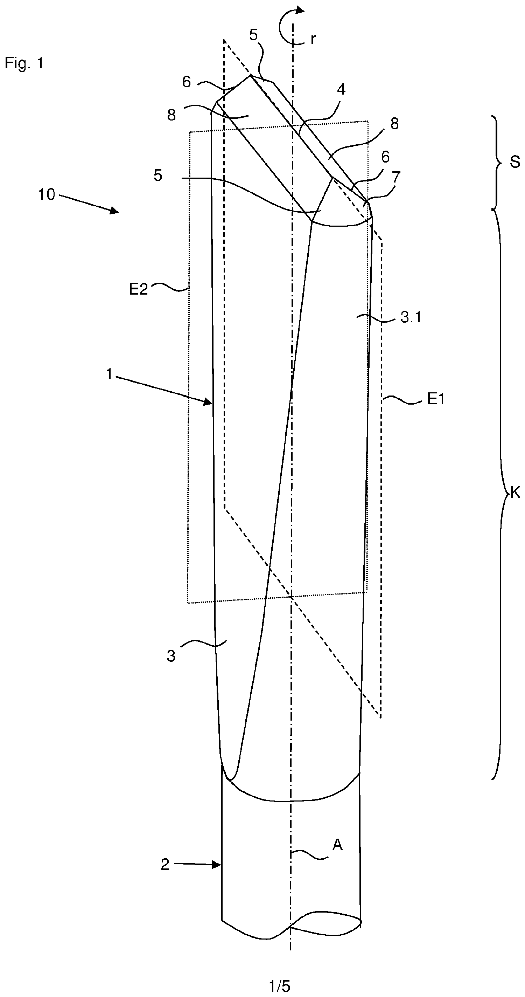

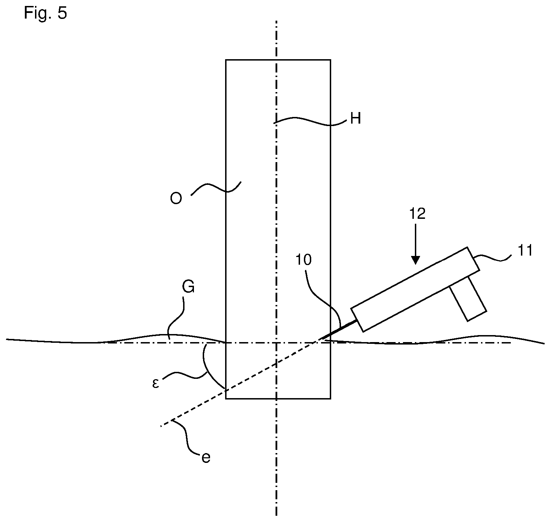

Das in **Fig. 1 bis 4** dargestellte Beispiel zeigt als erfindungsgemäße Vorrichtung eine Bohrnadel 10 mit einem spezifisch geformten Bohrnadelkopf 1, der besonders für Bohrwiderstandsmessungen mittels eines Bohrwiderstandsmessgeräts 12, wie in **Fig. 5** angedeutet, in einem Holzobjekt O in einem von 90° zur Objektachse H abweichenden Eindringwinkel ε geeignet ist. Es sind auch ein Bohrwiderstandsmessgerät 12 mit einer erfindungsgemäßen Bohrnadel 10 und dessen Verwendung zur Beschaffenheitsuntersuchung von Holzobjekten O Gegenstände der Erfindung.

Die spezifische Form des Bohrnadelkopfes 1 einer erfindungsgemäßen Bohrnadel 10, wie sie in **Figuren 1 bis** 4 zu sehen ist, beruht auf einem abgeflachten Keilabschnitt K mit anschließendem Schneidenabschnitt S an einem Ende des zylindrischen Bohrnadelschafts 2. Der Keilabschnitt K wird durch zwei in entgegen gesetzte Richtungen liegende Keilflächen 3 definiert, die seitlich über zwei entsprechende abgerundete Flankensegmente 3.1 ergänzt werden. Der Schneidenabschnitt S besteht aus zwei an einer Kopfkante 4 angrenzenden Schneidflächen 8, die sich an die Keilflächen 3 anschließen und mit diesen jeweils eine Keilabschnittskante 9 ausbilden. Die Kopfkante 4 verläuft im rechten Winkel zu einer Rotationsachse A des Bohrnadelschafts 2, und gestattet die Definition einer ersten Bezugsebene E1, die durch die Kopfkante 4 und die Rotationsachse A definiert wird. Eine zweite Bezugsebene E2 verläuft senkrecht, d. h. im rechten Winkel, zur Kopfkante 4 und ebenfalls durch die Rotationsachse A.

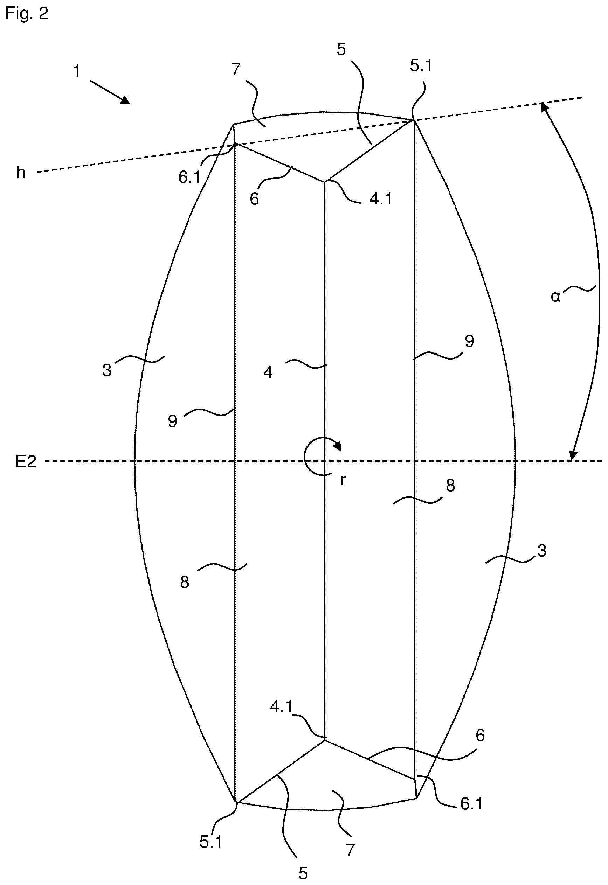

Im Schneidenabschnitt S weist der Bohrnadelkopf 1 seitlich an die zwei Schneidflächen 8 angrenzend jeweils eine Freischlifffläche 7 auf, die - wie besonders gut in der Draufsicht in **Fig. 2** zu erkennen ist - mit jeweils einer der Schneidflächen 8 eine in Bezug auf eine in Rotationsrichtung r vorlaufende Schneidkante 5 bildet, die sich von einer Kopfecke 4.1 an der Kopfkante 4 zu einer Schneidkantenecke 5.1 an der Keilfläche 3 erstreckt. Mit der jeweils anderen der Schneidflächen 8 bildet jede Freischlifffläche 7 eine in Bezug auf die Rotationsrichtung r nachlaufende Nebenkante 6, die sich von der Kopfecke 4.1 an der Kopfkante 4 zu einer Nebenkantenecke 6.1 an der Keilfläche 3 erstreckt. Sowohl die beiden Schneidkanten 5 als auch die beiden Nebenkanten 6 und die dadurch begrenzten Freiflächen 7 können durch Rotation um 180° um die Rotationsachse A ineinander abgebildet werden und sind somit in der Draufsicht punktsymmetrisch in Bezug auf die Rotationsachse. Der Freischliffwinkel α, den die virtuelle Gerade h, die durch die Schneidkantenecke 5.1 und die Nebenkantenecke 6.1 verläuft, mit der Ebene E2 aufspannt, die sich durch die Rotationsachse A senkrecht zu der Kopfkante 4 erstreckt, beträgt im dargestellten Beispiel 8° und kann erfindungsgemäß in einem Bereich von 6° bis 10° liegen. Damit ist die nachlaufende Nebenkante 6 kürzer als die vorlaufende Schneidkante 5, sodass die Freischlifffläche 7 die Form eines unregelmäßigen Vierecks mit einer abgerundeten Seite aufweist, die der Kante zum gerundeten Flankensegment 3.1 entspricht. Neben der Kopfecke 4.1, der Schneidkantenecke 5.1 und der Nebenkantenecke 6.1 weist die Freischlifffläche 7 eine weitere Ecke (nicht bezeichnet) auf, die benachbart zu der Nebenkantenecke 6.1 an der Kante zwischen Keilfläche 3 und dem Flankensegment 3.1 vorliegt.

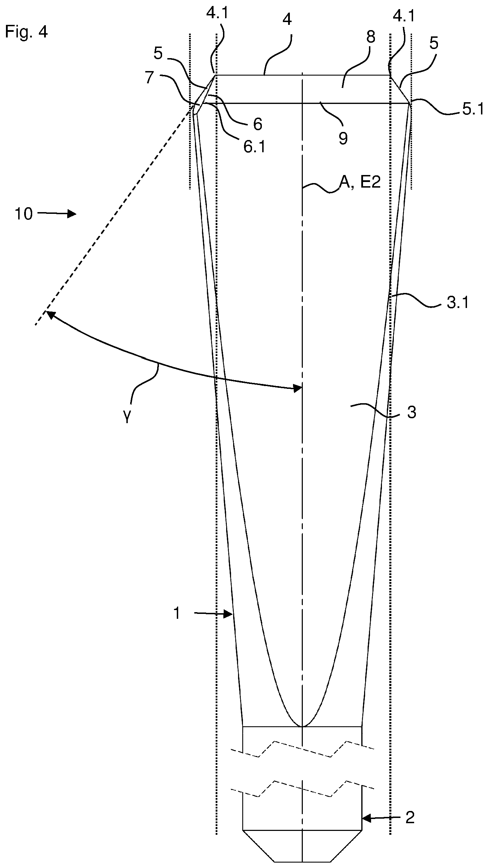

**Fig. 4** zeigt den Schneidenwinkel γ einer erfindungsgemäßen Bohrnadel 10 in einer beispielhaften Ausführungsform. Der Schneidenwinkel γ ist zwischen der Schneidkante 5 und der Rotationsachse A bzw. der Ebene E2 definiert und beträgt im gezeigten Beispiel 30°, kann aber abweichend davon in einem Bereich von 25° bis 35° liegen. Ferner ist in **Fig. 4** durch die gepunkteten Linien erkennbar, dass die Kopfkante 4 einen Rotationsdurchmesser bereitstellt, der breiter ist als ein Durchmesser des Bohrnadelschafts 2, aber kleiner ist als der Rotationsdurchmesser, den der Bohrnadelkopf 1 in dem Übergangsbereich des Keilabschnitts K mit dem Schneidenabschnitt S, d. h. im Bereich der abgerundeten Kante der Freischlifffläche 7 zu dem Flankensegment 3.1, aufweist.

Im dargestellten Beispiel von **Fig. 1 bis 4** weist die Kopfkante 4 eine Breite von 2,20 mm auf, während die maximale Breite des Bohrnadelkopfs 1 im Bereich der abgerundeten Kante der Freischlifffläche 7 zu dem Flankensegment 3.1 am Übergangsbereich des Keilabschnitts K mit dem Schneidenabschnitt S bei 2,75 mm liegt. Die Höhe des Schneidenabschnitts S in axialer Richtung zwischen Kopfkante 4 und Keilabschnittskante 9 beträgt hier 0,35 mm.

Abweichend von diesen bevorzugten Abmessungen kann eine erfindungsgemäße Bohrnadel 10 eine Breite der Kopfkante 4 in einem Bereich von 2,00 bis 2,50 mm aufweisen, wobei die maximale Breite des Bohrnadelkopfs 1 im Bereich der abgerundeten Kante der Freischlifffläche 7 zum Flankensegment 3.1 in einem Bereich von 2,25 bis 3,00 mm liegen kann, unter der Bedingung, dass die Breite der Kopfkante 4 kleiner ist als die maximale Breite. Die Höhe des Schneidenabschnitts S kann in einem Bereich von 0,30 bis 0,40 mm liegen.

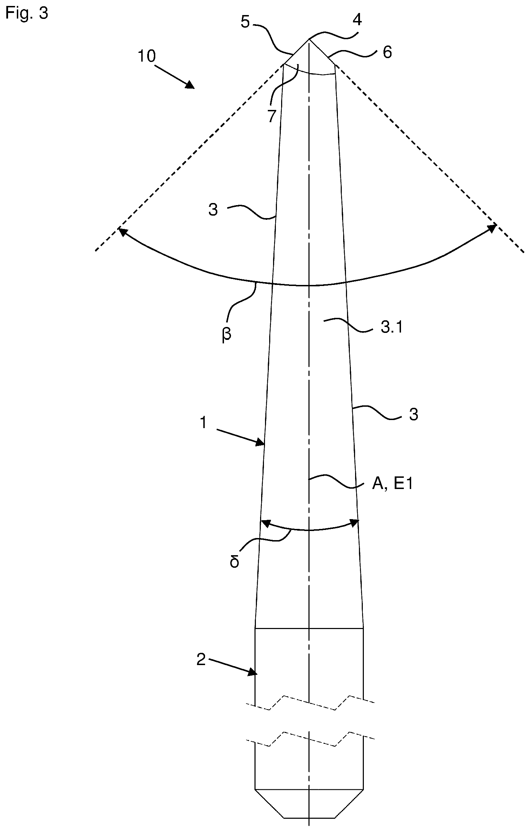

In der in **Fig. 3** dargestellten Seitenansicht sind der Winkel δ zwischen den beiden Keilflächen 3 und der Kopfkantenwinkel β zwischen den beiden Schneidflächen 8 dargestellt, wobei zu erkennen ist, dass der Winkel δ zwischen den beiden Keilflächen 3, der hier mit 15° innerhalb des erfindungsgemäßen Bereichs von 10° bis 20° liegt, deutlich kleiner ist als der Kopfkantenwinkel β, der im dargestellten Beispiel 90° beträgt, erfindungsgemäß aber grundsätzlich in einem Bereich von 85° bis 95° liegen kann.

Nach einer bevorzugten Ausführungsform kann die Bohrnadel 10 aus einem Stahl gefertigt sein und eine Hartverchromung als Oberflächenbeschichtung mit einer Schichtdicke in einem Bereich von 8 bis 12 µm aufweisen.

In **Fig. 5** sind ein erfindungsgemäßes Bohrwiderstandsmessgerät 12 und dessen Verwendung zur Beschaffenheitsuntersuchung eines Holzobjekts O angedeutet. Das Bohrwiderstandsmessgerät 12 weist eine erfindungsgemäße Bohrnadel 10, beispielsweise die in **Fig. 1 bis 4** dargestellte Bohrnadel 10, und ein Handgerät 11 auf, dessen Details zwar nicht dargestellt und bezeichnet sind, die aber zumindest eine Antriebsvorrichtung mit einem Bohrfutter, in dem die Bohrnadel 10 eingespannt ist, und optional eine Führungsvorrichtung zum geführten Einführen der Bohrnadel 10 in das zu untersuchende Holzobjekt O und/oder eine Bohrwiderstandserfassungseinrichtung aufweist.

Dieses Bohrwiderstandsmessgerät 12, das eine erfindungsgemäße Bohrnadel 10 aufweist, kann zur Beschaffenheitsuntersuchung eines Holzobjekts O verwendet werden, wobei beim Einführen der Bohrnadel 10 in das Holzobjekt O in einer axialen Vorschubrichtung e, die beliebig in Bezug auf eine Objektachse H oder eine Erdgleiche G wählbar ist, ein geradliniger Bohrkanal in der axialen Vorschubrichtung e erzeugt wird, indem ein Abdriften der Bohrnadel 10 durch die spezifische Ausbildung des Bohrnadelkopfes 1 vermieden wird. Bevorzugt kann das Holzobjekt O einen Abschnitt unter der Erdgleiche G aufweisen, der mit dem Bohrwiderstandsmessgerät 12 untersucht werden soll. Dazu kann sich der Bohrkanal von einer Eindringstelle oberhalb der Erdgleiche G in den Abschnitt unterhalb der Erdgleiche G erstrecken, indem ein Eindringwinkel ε zwischen der axialen Vorschubrichtung e und der Erdgleiche G in einem Bereich von 15° bis 45° betragen kann. Der Eindringwinkel ε, um einen zu überprüfenden Bereich des Objekts O unterhalb der Erdgleiche G zu erreichen, hängt nicht nur von der Höhe der Eindringstelle in das Objekt O über der Erdgleiche G ab, sondern auch von dem Durchmesser des zu untersuchenden Objekts O.

### BEZUGSZEICHENLISTE

- 1: Bohrnadelkopf
- 2: Schaft
- 3: Keilfläche
- 3.1: Flankensegment
- 4: Kopfkante
- 4.1: Kopfecke
- 5: Schneidkante
- 5.1: Schneidkantenecke
- 6: Nebenkante
- 6.1: Nebenkantenecke
- 7: Freischlifffläche
- 8: Schneidfläche
- 9: Keilabschnittskante
- 10: Bohrnadel
- 11: Handgerät
- 12: Bohrwiderstandsmessgerät
- A: Rotationsachse
- E1: Ebene durch Rotationsachse und durch Kopfkante
- E2: Ebene durch Rotationsachse und senkrecht zur Kopfkante
- G: Erdgleiche
- H: Objektachse
- K: Abgeflachter Keilabschnitt
- O: Holzobjekt
- S: Schneidenabschnitt
- e: Vorschubrichtung
- h: Virtuelle Gerade
- r: Rotationsrichtung
- α: Freischliffwinkel
- β: Kopfkantenwinkel
- γ: Schneidenwinkel
- δ: Keilflächenwinkel
- ε: Eindringwinkel

## Patentansprüche

1. Bohrnadel (10) mit einem Bohrnadelkopf (1), der an einem Ende eines Bohrnadelschafts (2) einen abgeflachten Keilabschnitt (K) mit zwei Keilflächen (3) und einen Schneidenabschnitt (S) mit zwei Schneidflächen (8) aufweist, die jeweils an eine der Keilflächen (3) anschließen und an einer Kopfkante (4) aneinander angrenzen, die im rechten Winkel zu einer Rotationsachse (A) des Bohrnadelschafts (2) verläuft, **dadurch gekennzeichnet, dass**
der Bohrnadelkopf (1) seitlich an die zwei Schneidflächen (8) angrenzend jeweils eine Freischlifffläche (7) aufweist, die
- mit jeweils einer der Schneidflächen (8) eine in Bezug auf eine Rotationsrichtung (r) vorlaufende Schneidkante (5) bildet, die sich von einer Kopfecke (4.1) an der Kopfkante (4) zu einer Schneidkantenecke (5.1) an der Keilfläche (3) erstreckt, und
- mit der jeweils anderen der Schneidflächen (8) eine in Bezug auf die Rotationsrichtung (r) nachlaufende Nebenkante (6) bildet, die sich von der Kopfecke (4.1) an der Kopfkante (4) zu einer Nebenkantenecke (6.1) an der Keilfläche (3) erstreckt, wobei ein Freischliffwinkel (α), den eine virtuelle Gerade (h), die durch die Schneidkantenecke (5.1) und die Nebenkantenecke (6.1) verläuft, mit einer Ebene (E2) aufspannt, die sich senkrecht zu der Kopfkante (4) erstreckt, wobei die Rotationsachse (A) in der Ebene (E2) liegt, in einem Bereich von 6° bis 10° liegt.

2. Bohrnadel (10) nach Anspruch 1,
**dadurch gekennzeichnet, dass**
ein Schneidenwinkel (γ), den die Schneidkante (5) in Bezug auf die Rotationsachse (a) aufspannt, in einem Bereich von 25° bis 35° liegt und bevorzugt 30° beträgt.

3. Bohrnadel (10) nach Anspruch 1 oder 2,
**dadurch gekennzeichnet, dass**
die Kopfkante (4) einen ersten Rotationsdurchmesser bereitstellt, der breiter ist als ein Durchmesser des Bohrnadelschafts (2), wobei bevorzugt der Bohrnadelkopf (1) in einem Übergangsbereich des Keilabschnitts (K) mit dem Schneidenabschnitt (S) einen zweiten Rotationsdurchmesser aufweist, der größer ist als der erste Rotationsdurchmesser;
und/oder
ein Winkel (δ) zwischen den beiden Keilflächen (3) kleiner ist als ein Kopfkantenwinkel (β) zwischen den beiden Schneidflächen (8).

4. Bohrnadel (10) nach Anspruch 3,
**dadurch gekennzeichnet, dass**
der Kopfkantenwinkel (β) in einem Bereich von 85° bis 95° liegt und bevorzugt 90° beträgt.

5. Bohrnadel (10) nach zumindest einem der Ansprüche 1 bis 4,
**dadurch gekennzeichnet, dass**
der Freischliffwinkel (α) 8° beträgt.

6. Bohrnadel (10) nach zumindest einem der Ansprüche 1 bis 5,
**dadurch gekennzeichnet, dass**
die Bohrnadel (10) aus einem Stahl gefertigt ist und zumindest der Bohrnadelkopf (1) eine Oberflächenbeschichtung aufweist.

7. Bohrnadel (10) nach Anspruch 6,
**dadurch gekennzeichnet, dass**
die Oberflächenbeschichtung eine Hartverchromung ist, bevorzugt mit einer Schichtdicke in einem Bereich von 8 bis 12 µm.

8. Bohrnadel (10) nach zumindest einem der Ansprüche 1 bis 7,
**dadurch gekennzeichnet, dass**
- eine Breite der Kopfkante (4) in einem Bereich von 2,00 bis 2,50 mm liegt und bevorzugt 2,20 mm beträgt; und
- eine Breite des Bohrnadelkopfs (1) in einem Übergangsbereich des Keilabschnitts (K) mit dem Schneidenabschnitt (S) in einem Bereich von 2,25 bis 3,00 mm liegt und bevorzugt 2,75 mm beträgt; und
- eine Höhe des Schneidenabschnitts (S) in einem Bereich von 0,30 bis 0,40 mm liegt und bevorzugt 0,35 mm beträgt.

9. Bohrwiderstandsmessgerät (12), das eine Bohrnadel (10) und ein Handgerät (11) aufweist, das eine Antriebsvorrichtung mit einem Bohrfutter, in dem die Bohrnadel (10) eingespannt ist, und optional eine Führungsvorrichtung zum geführten Einführen der Bohrnadel (10) in ein zu untersuchendes Holzobjekt (O) und/oder eine Bohrwiderstandserfassungseinrichtung aufweist,
**dadurch gekennzeichnet, dass**
die Bohrnadel (10) eine Bohrnadel (10) nach zumindest einem der Ansprüche 1 bis 8 ist.

10. Verwendung eines Bohrwiderstandsmessgeräts (12) nach Anspruch 9 mit einer Bohrnadel (10) nach zumindest einem der Ansprüche 1 bis 8 zur Beschaffenheitsuntersuchung eines Holzobjekts (O), wobei beim Einführen der Bohrnadel (10) in das Holzobjekt (O) in einer axialen Vorschubrichtung (e), die beliebig in Bezug auf eine Objektachse (H) wählbar ist, ein geradliniger Bohrkanal in der axialen Vorschubrichtung (e) erzeugt wird.

## Claims

1. A drilling needle (10) with a drilling needle head (1) that, at one end of a drilling needle shank (2), has a flattened wedge portion (K) with two wedge faces (3) and a cutter portion (S) with two cutting faces (8), which in each case adjoin one of the wedge faces (3) and adjoin one another at a top edge (4) that runs at a right angle to an axis of rotation (A) of the drilling needle shank (2),
**characterized in that**
the drilling needle head (1) has, on the side adjoining the two cutting faces (8), in each case a relief face (7) that
- with in each case one of the cutting faces (8), forms a cutting edge (5) that leads with respect to a direction of rotation (r) and that extends from a top corner (4.1) at the top edge (4) to a cutting edge corner (5.1) at the wedge face (3), and
- with the respective other one of the cutting faces (8), forms a secondary edge (6) that trails with respect to the direction of rotation (r), which extends from the top corner (4.1) at the top edge (4) to a secondary edge corner (6.1) on the wedge face (3),
wherein a relief angle (α), which a virtual straight line (h) that runs through the cutting edge corner (5.1) and the secondary edge corner (6.1) spans with a plane (E2) that extends through the axis of rotation (A) perpendicular to the top edge (4), lies in a range from 6° to 10°.

2. The drilling needle (10) according to claim 1,
**characterized in that**
a cutting edge angle (γ), which the cutting edge (5) spans with respect to the axis of rotation (a), lies in a range from 25° to 35° and is preferably 30°.

3. The drilling needle (10) according to claim 1 or 2,
**characterized in that**
the top edge (4) provides a first diameter of rotation which is wider than a diameter of the drilling needle shank (2), wherein preferably the drilling needle head (1) in a transition region of the wedge portion (K) with the cutter portion (S) has a second rotation diameter that is larger than the first diameter of rotation;
and/or
an angle (δ) between the two wedge faces (3) is smaller than a top edge angle (β) between the two cutting faces (8).

4. The drilling needle (10) according to claim 3,
**characterized in that**
the top edge angle (β) lies in a range from 85° to 95° and is preferably 90°.

5. The drilling needle (10) according to at least any one of the claims 1 to 4, **characterized in that**
the relief angle (α) is 8°.

6. The drilling needle (10) according to at least any one of the claims 1 to 5, **characterized in that**
the drilling needle (10) is made of a steel and at least the drilling needle head (1) has a surface coating.

7. The drilling needle (10) according to claim 6,
**characterized in that**
the surface coating is a hard chrome plating, preferably with a layer thickness in a range of 8 to 12 µm.

8. The drilling needle (10) according to at least any one of the claims 1 to 7, **characterized in that**
- a width of the top edge (4) lies in a range of 2.00 to 2.50 mm and is preferably 2.20 mm; and
- a width of the drilling needle head (1) in a transition region of the wedge portion (K) with the cutter portion (S) lies in a range of 2.25 to 3.00 mm and is preferably 2.75 mm; and
- a height of the cutter portion (S) lies in a range of 0.30 to 0.40 mm and is preferably 0.35 mm.

9. A drilling resistance measuring device (12) that has a drilling needle (10) and a hand-held device (11) that has a drive device with a drill chuck in which the drilling needle (10) is clamped, and optionally a guide device for guided insertion of the drilling needle (10) into a wooden object (O) to be examined and/or a drilling resistance detection device,
**characterized in that**
the drilling needle (10) is a drilling needle (10) according at least any one of the claims 1 to 8.

10. A use of a drilling resistance measuring device (12) according to claim 9 with a drilling needle (10) according at least one of the claims 1 to 8 for examining properties of a wooden object (O), wherein when the drilling needle (10) is inserted into the wooden object (O) in an axial feed direction (e), which can be selected as desired with respect to an object axis (H), a straight-line drilling channel is created in the axial feed direction (e).

## Revendications

1. Aiguille de perçage (10) avec une tête d'aiguille de perçage (1) qui présente, à une extrémité de la tige de l'aiguille de perçage (2), une section en coin aplatie (K) avec deux surfaces en coin (3) et une section de coupe (S) avec deux surfaces de coupe (8) qui se raccordent chacune à l'une des surfaces en coin (3) et qui sont adjacentes l'une à l'autre au niveau d'un bord supérieur (4) qui est perpendiculaire à un axe de rotation (A) de la tige de l'aiguille de perçage (2),
**caractérisée en ce que**
la tête d'aiguille de perçage (1) présente latéralement, de manière adjacente aux deux surfaces de coupe (8), respectivement une surface de dégagement (7) qui
- forme, avec respectivement l'une des surfaces de coupe (8), un bord de coupe (5) en amont par rapport à une direction de rotation (r), qui s'étend d'un coin supérieur (4.1) sur le bord supérieur (4) jusqu'à un coin du bord de coupe (5.1) sur la surface en coin (3), et
- forme, avec l'autre des surfaces de coupe (8), un bord secondaire (6) en aval par rapport à la direction de rotation (r), qui s'étend du coin supérieur (4.1) sur le bord supérieur (4) jusqu'à un coin du bord secondaire (6.1) sur la surface en coin (3), un angle de dépouille (α) formé par une droite virtuelle (h) passant par le coin du bord de coupe (5. 1) et le coin du bord secondaire (6.1), avec un plan (E2) perpendiculaire au bord supérieur (4), se situant dans une plage de 6° à 10°, l'axe de rotation (A) étant situé dans le plan (E2).

2. Aiguille de perçage (10) selon la revendication 1,
**caractérisée en ce que**
un angle de coupe (γ), formé par le bord de coupe (5) par rapport à l'axe de rotation (a), se situe dans une plage de 25° à 35° et est de préférence de 30°.

3. Aiguille de perçage (10) selon la revendication 1 ou 2,
**caractérisée en ce que**
le bord supérieur (4) fournit un premier diamètre de rotation qui est plus large qu'un diamètre de la tige de l'aiguille de perçage (2), la tête d'aiguille de perçage (1) présentant dans une zone de transition de la section en coin (K) avec la section de coupe (S) de préférence un deuxième diamètre de rotation qui est plus grand que le premier diamètre de rotation ;
et/ou
un angle (δ) entre les deux surfaces en coin (3) est plus petit qu'un angle du bord supérieur (β) entre les deux surfaces de coupe (8).

4. Aiguille de perçage (10) selon la revendication 3,
**caractérisée en ce que**
l'angle du bord supérieur (β) se situe dans une plage de 85° à 95° et est de préférence de 90°.

5. Aiguille de perçage (10) selon au moins l'une des revendications 1 à 4,
**caractérisée en ce que**
l'angle de dépouille (α) est de 8°.

6. Aiguille de perçage (10) selon la revendication 1 à 5,
**caractérisée en ce que**
l'aiguille de perçage (10) est fabriquée en acier et au moins la tête d'aiguille de perçage (1) présente un revêtement de surface.

7. Aiguille de perçage (10) selon la revendication 6,
**caractérisée en ce que**
le revêtement de surface est un chromage dur, de préférence avec une épaisseur de couche comprise dans une plage de 8 à 12 µm.

8. Aiguille de perçage (10) selon au moins l'une des revendications 1 à 7,
**caractérisée en ce que**
- une largeur du bord supérieur (4) se situe dans une plage de 2,00 à 2,50 mm et est de préférence de 2,20 mm ; et
- une largeur de la tête d'aiguille de perçage (1) dans une zone de transition de la section en coin (K) avec la section de coupe (S) se situe dans une plage de 2,25 à 3,00 mm et est de préférence de 2,75 mm ; et
- une hauteur de la section de coupe (S) se situe dans une plage de 0,30 à 0,40 mm et est de préférence de 0,35 mm.

9. Appareil de mesure de la résistance de perçage (12) comprenant une aiguille de perçage (10) et un appareil à main (11), lequel comprend un dispositif d'entraînement avec un mandrin de perçage, dans lequel l'aiguille de perçage (10) est serrée, et en option un dispositif de guidage pour l'introduction guidée de l'aiguille de perçage (10) dans un objet en bois (O) à analyser et/ou un dispositif de détection de la résistance de perçage,
**caractérisé en ce que**
l'aiguille de perçage (10) est une aiguille de perçage (10) selon au moins l'une des revendications 1 à 8.

10. Utilisation d'un appareil de mesure de la résistance de perçage (12) selon la revendication 9, avec une aiguille de perçage (10) selon au moins l'une des revendications 1 à 8, pour l'analyse des caractéristiques d'un objet en bois (O), un canal de perçage rectiligne étant généré dans la direction d'avance axiale (e) lors de l'introduction de l'aiguille de perçage (10) dans l'objet en bois (O) dans une direction d'avance axiale (e) qui peut être sélectionnée de manière quelconque par rapport à un axe d'objet (H).
